# EUROPÄISCHE PATENTANMELDUNG

(11) **EP 1 364 640 A1**
(43) Veröffentlichungstag der Anmeldung: **26.11.2003**
(21) Anmeldenummer: 03011451.6
(22) Anmeldetag: 20.05.2003
(51) Int. Cl.: A61K 7/48, A61K 33/14

(54) **Pharmazeutische Zusammensetzung mit hautentquellender Wirkung**

(30) Priorität: 24.05.2002 DE 10223221
(71) Anmelder: Sebapharma GmbH & Co. KG, 56154 Boppard-Bad Salzig (DE)
(72) Erfinder: Gottfreund, Joachim, Dr., 56154 Boppard (DE); Meyer, Thomas, Dipl.-Ing., 56281 Emmelshausen (DE)
(74) Vertreter: Becker Kurig Straus

(57) **Zusammenfassung**

Die vorliegende Erfindung betrifft eine pharmazeutische Zusammensetzung mit hautentquellender Wirkung, die aus mindestens einem Alkali- und/oder Erdalkalichlorid als Wirkstoff und einem pharmazeutisch akzeptablen Träger als Grundlage besteht, der ausgewählt ist aus der Gruppe, bestehend aus einem flüssigen Basismaterial, einem pH-Regulator, und gegebenenfalls einer Ölkomponente. Die pharmazeutische Zusammensetzung gemäß der vorliegenden Erfindung wird zur Herstellung eines topischen Präparats zur Prophylaxe und/oder Behandlung von Hautekzemen, -rötungen, -trockenheit.

## Beschreibung

Die vorliegende Erfindung betrifft eine pharmazeutische Zusammensetzung mit hautentquellender Wirkung, insbesondere eine pharmazeutische Zusammensetzung mit hautentquellender Wirkung, die als Wirkstoff mindestens ein Alkali- und/oder Erdalkalichlorid, insbesondere Natriumchlorid, umfasst. Die erfindungsgemäße pharmazeutische Zusammensetzung wird insbesondere zur Herstellung eines topischen Präparats zur Prophylaxe und/oder Behandlung von Hautekzemen, -rötungen, -trockenheit, -einrissen, -schuppungen und - juckreiz eingesetzt.

Eine intakte Hornschicht, d. h. eine epidermale Barriere, ist eine wesentliche Voraussetzung für die Schutzfunktion der Haut gegenüber äußeren Einflüssen. Der länger dauernde oder ständig wiederholte Kontakt mit Wasser führt zur Schädigung der epidermalen Barriere und der darunter liegenden Hautschichten [1], d. h. der lebenden Epidermis. Die gleiche Situation, verbunden mit einem Feuchtigkeits- und Wärmestau, stellt sich ein, wenn über einen längeren Zeitraum durch Latex-Handschuhe u. ä. oder Chemikalien ein Gasaustausch zwischen Haut und Umgebung unterbunden wird, die Hornschicht quillt, was als Mazeration der Haut bezeichnet wird [2]. Durch die Beeinträchtigung der Barrierefünktion kommt es zur stärkeren Einwirkung von äußeren Stoffen auf die Haut und dem Verlust körpereigener Stoffe wie Wasser und/oder Elektrolyte aus der Haut, d. h. es führt zu einem transepidermalen Wasserverlust [3]. Es entsteht ein Abnutzungsekzem, das als klinisches Bild Rötung, Trockenheit, Schuppung, Einrisse, Juckreiz der Haut zeigt, und das bei mangelndem Hautschutz in ein chronisches Ekzem übergehen kann.

Darüber hinaus können potentielle Allergene leicht in die Haut eindringen und zur Sensibilisierung führen [4]. Die Entstehung von allergischen Kontaktekzemen kann durch das Abnutzungsekzem gefördert werden.

Für dieses Problem werden zur Zeit Präparate angeboten, die die geschädigte Barriere wieder herstellen sollen oder die Haut vor dem Kontakt mit externen Noxen schützen [5]. Für verschiedene Berufsgruppen wie Friseure u. ä. sind daher bienenwachshaltige oder fluorkohlenwasserstoffhaltige Hautschutzpräparate empfohlen worden [6]. Die Wirkung dieser Präparate besteht darin, dass sie eine chemische oder physiologische Barriere auf der Haut herstellen, die verhindert, dass Wasser oder andere Irritationen an sie gelangen. Sie haben aber keine entquellende Wirkung.

Auch durch Emulsionen, die Öl-in-Wasser oder Wasser-in-Öl-Emulsionen darstellen, werden nur die in Defizit geratenen Inhaltsstoffe der Haut wieder aufgefüllt, z. B. hautfeuchtigkeitssteigernde und fettende Verbindungen. Dabei können in O/W-Systemen sogar durch die eingesetzten Emulgatoren zusätzliche desikierende Wirkungen erzeugt werden [7].

Diese kosmetisch akzeptablen Präparationen sind daher wirkungsloser als W/O-Lotionen oder -Cremes, wobei diese wiederum den Nachteil haben, dass sie auf gequollener Haut nicht applizierbar sind, da sich auf der Haut kein zusammenliegender Fettfilm ausbilden kann. Durch derartige Präparate wird die Hornschicht nicht in den ursprünglichen Zustand zurückgerührt, d. h. entgequollen.

Bisher gibt es kein Produkt, dass durch Entquellung der gequollenen Haut sie in ihren Ausgangszustand wieder zurückführt.

Eine entquellend wirkende Präparation zeichnet sich dadurch aus, dass der transepidermale Wasserverlust der Haut gesenkt und die Hautrauhigkeit reduziert wird. Wünschenswert ist, dass nach Applikation des Produktes die Hautfeuchtigkeit den Ausgangswert nach möglichst kurzer Zeit wieder erreicht [8].

Bei der Anwendung von Natriumchlorid auf Haut war bisher nur der entgegengesetzte Effekt, nämlich eine hydratisierende Wirkung oder sogar eine aufweichende Wirkung durch Natriumchlorid beschrieben [9].

DE 696 21 213 T2 beschreibt eine topische Gelzusammensetzung zur Hydratisierung der Haut und/oder zur Behandlung von empfindlicher Haut, die einen Elektrolyten, wie ein beispielsweise Natriumchlorid, enthält. Diese Zusammensetzung umfasst neben einem Elektrolyten, eine wässerige Phase und einen pharmazeutischen und/oder dermatologischen Wirkstoff und als Gelbildner ausschließlich Cetylhydroxyethylcellulose, welches die Zusammensetzung stabilisieren soll.

Elektrolyten-haltige Zusammensetzungen werden weiterhin als Träger für Haarwaschmittel eingesetzt, um Konditioniermittel und Antischuppenmittel langzeitig in Suspension zu halten. DE 697 04 224 T2 beschreibt eine derartige Zusammensetzung, die mindestens ein anionisches Tensid, mindestens ein nichtionisches amphoteres Cotensid, mindestens ein verdickendes Polyacrylamid und mindestens einen Elektrolyten enthält. Als Elektrolyt können Alkalimetallsalze und Erdalkalisalze verwendet werden. Entscheidend, um nicht wasserlösliche Konditioniermittel in Suspension zu halten, ist die Viskosität des Trägers. DE 697 03 942 T2 beschreibt die eine Variante der Zusammensetzung gemäß DE 697 04 224 T2, bei der alkoxyliertes Silicon anstelle von Polyacrylamid verwendet wurde, um die gewünschte Viskosität zu erzielen. Eine Wirkung auf die Haut von derartigen Zusammensetzungen wird nicht beschrieben.

Es besteht eine Nachfrage nach einer pharmazeutischen Zusammensetzung, die gequollene Haut durch Entquellung in ihren Ausgangszustand zurückführt. Eine derartige entquellend wirkende Zusammensetzung muss bewirken, dass der transepidermale Wasserverlust der Haut gesenkt und gleichzeitig die Rauhigkeit der Haut reduziert wird, sodass nach Applikation der Zusammensetzung nach einer möglichst kurzen Zeitdauer die Hautfeuchtigkeit ihren Ausgangszustand erreicht.

Die Aufgabe der vorliegenden Erfindung ist es daher, eine Zusammensetzung bereitzustellen, die entquellend auf Haut wirkt.

Die Aufgabe wird erfindungsgemäß durch die Bereitstellung einer pharmazeutischen Zusammensetzung mit hautentquellender Wirkung gelöst, die aus mindestens einem Alkaliund/oder Erdalkalichlorid als Wirkstoff und einem pharmazeutisch akzeptablen Träger als Grundlage besteht, der ausgewählt ist aus der Gruppe, bestehend aus wenigstens einem flüssigen Basismaterial, wenigstens einem pH-Regulator, und gegebenenfalls wenigstens einer Ölkomponente.

In einer bevorzugten Ausführungsform ist das mindestens eine Alkali- und/oder Erdalkalichlorid ausgewählt aus der Gruppe, bestehend aus Natriumchlorid, Magnesiumchlorid, Calciumchlorid, Kaliumchlorid und deren Mischungen, wobei Natriumchlorid besonders bevorzugt ist. Weitere bevorzugte Ausführungsformen der vorliegenden Erfindung umfassen Mischungen aus Natriumchlorid und Calciumchlorid in einem Verhältnis von 10 : 1 bis 1 : 1, Mischungen aus Natriumchlorid und Magnesiumchlorid in einem Verhältnis von 20 : 1 bis 1: 5 und Mischungen aus Magnesiumchlorid und Calciumchlorid in einem Verhältnis von 1 : 1. Besonders bevorzugt wird Natriumchlorid in einer Konzentration von 5 bis 20 Gew.-%, insbesondere 5 bis 15 Gew.-%, in der pharmazeutischen Zusammensetzung, basierend auf der Gesamtzusammensetzung, verwendet.

Selbstverständlich können die Alkali- und/oder Erdalkalichloride auch in ihren Hydratformen eingesetzt werden. Weiterhin können die vorstehenden Salze entweder in technisch reiner Form oder als Rohstoff eingesetzt werden, d. h. sie können Verunreinigungen und dem Fachmann bekannte Zusätze, beispielsweise Spuren von Metallionen wie Bariumionen, Eisenoxid und dergleichen, Iodide, Bromide, Chlorate, Nitrate, Phosphate oder Sulfate enthalten.

Der Träger der erfindungsgemäßen pharmazeutischen Zusammensetzung besteht aus wenigstens einem flüssigen Basismaterial, wenigstens einem pH-Regulator, und gegebenenfalls wenigstens einer Ölkomponente, um dem Benutzer ein angenehmes Hautgefühl bei dem Anwenden der pharmazeutischen Zusammensetzung gemäß der Erfindung zu verschaffen.

Erfindungsgemäß umfasst das flüssige Basismaterial Wasser und/oder Alkohole mit 2 bis 4 Kohlenstoffatomen und/oder deren Mischungen. In bevorzugten Ausführungsformen ist das flüssige Basismaterial Ethanol, das aus Kostengründen, beispielsweise mit 1 % Diethylphthalat, vergällt sein kann, oder Isopropanol, welche die Trocknungszeit von feucht formulierten Produkten beschleunigen und einen angenehmen kühlenden Effekt bewirken. Weiterhin können Butanol, n-Propanol, sec-Butanol und Isobutanol verwendet werden. Es können auch Mischungen der vorstehenden Alkohole eingesetzt werden, sowie Mischungen aus einem oder mehreren Alkoholen mit Wasser. In dem letzteren Fall liegt das Gewichtsverhältnis von Alkohol zu Wasser vorzugsweise zwischen 1:5 und 4:5.

Zum Einstellen eines optimalen pH-Werts enthält die pharmazeutische Zusammensetzung Milchsäure oder ein Salz davon, Zitronensäure oder ein Salz davon und deren Mischungen, wobei die Salze insbesondere Alkali- und Erdalkalisalze umfassen, oder Alkali wie beispielsweise Natronlauge. Die pharmazeutische Zusammensetzung weist vorzugsweise einen pH-Wert auf, der im Bereich von leicht sauer bis neutral liegt, d. h. von 4,0 bis 7,0. Vorzugsweise ist der pH-Wert der erfindungsgemäßen pharmazeutischen Zusammensetzung 5,5. Der Anteil des zugesetzten pH-Regulators liegt vorzugsweise im Bereich von 5 bis 20 Gew.-%. Er ist besonders bevorzugt 15 Gew.-%.

Die Ölkomponente kann aus mehreren Stoffklassen ausgewählt werden. Die Ölkomponente umfasst beispielsweise Ester aus gesättigten und/oder ungesättigten, verzweigten und/oder unverzweigten Alkoholen einer Kettenlänge von 3 bis 30 Kohlenstoffatomen und gesättigten und/oder ungesättigten, verzweigten und/oder unverzweigten Alkancarbonsäuren einer Kettenlänge von 3 bis 30 Kohlenstoffatomen und/oder aromatischen Carbonsäuren. Bevorzugte Esteröle umfassen Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleylerucat, Erucyloleat, Erucylerucat sowie synthetische, halbsynthetische und natürliche Gemische solcher Ester wie z. B. Jojobaöl.

Weiterhin können als Ölkomponente Kohlenwasserstoffe oder Kohlenwasserstoffwachse, verwendet werden. Als Beispiele seien Paraffinöl, Squalan und Squalen genannt.

Weiterhin eignen sich gesättigte und/oder ungesättigte, verzweigte und/oder unverzweigte Alkohole, beispielsweise Octyldodecanol, oder Dialkylether, beispielsweise Dicaprylylether, als Ölkomponente.

In einer bevorzugten Ausführungsform werden Fettsäuretriglyceride als Ölkomponente verwendet, namentlich Triglycerinester gesättigter und/oder ungesättigter, verzweigter und/oder unverzweigter Alkancarbonsäuren einer Kettenlänge von 8 bis 24, vorzugsweise 12 bis 18 Kohlenstoffatomen. Die Ölkomponente kann hierbei ein synthetisches, halbsynthetisches oder natürliches Öl, beispielsweise Olivenöl, Sonnenblumenöl, Sojaöl, Erdnussöl, Rapsöl, Mandelöl, Palmöl, Kokosöl, Nerzöl oder Rizinusöl und dergleichen, sein.

Weiterhin kommen im Sinne der vorliegenden Erfindung Silikonöle als Ölkomponente in Betracht, die vorzugsweise linear oder cyclisch sind. Die verwendeten Silikonöle sind vorzugsweise Dialkyl- und Alkylarylsiloxane. In bevorzugten Ausführungsformen wird als Silikonöl Octamethylcyclotetrasiloxan, Hexamethylcyclotrisiloxan, Polydimethylsiloxan und Poly(methylphenylsiloxan) verwendet. Octamethylcyclotetrasiloxan wird besonders bevorzugt verwendet. Vorzugsweise umfasst eine Ölkomponente, die ein Silikonöl oder eine Mischung aus Silikonölen enthält, einen zusätzlichen Gehalt an einer weiteren Ölkomponente.

Selbstverständlich können erfindungsgemäß nämlich auch Mischungen der vorstehend genannten Öle, Fette und Wachse bzw. Stoffen und Verbindungen als Ölkomponente der vorliegenden Erfindung eingesetzt werden.

Bei Verwendung wenigstens einer Ölkomponente liegt der Ölkomponenten-Anteil im Bereich von 0,1 bis 20 Gew.-%, basierend auf der Gesamtzusammensetzung.

In bevorzugten Ausführungsformen besteht die Ölkomponente gemäß der vorliegenden Erfindung aus Mischungen aus C₁₂₋₁₅- Alkylbenzoat und 2-Ethylhexylisostearat, Mischungen aus C₁₂₋₁₅-Alkylbenzoat und Isotridecylisononanoat oder Mischungen aus C₁₂₋₁₅-Alkylbenzoat, 2-Ethylhexylisostearat und Isotridecylisononanoat.

In weiteren bevorzugten Ausführungsformen wird als Ölkomponente eine Mischung aus Octamethylcyclotetrasiloxan und Isotridecylisononanoat oder eine Mischung aus Octamethylcyclotetrasiloxan und 2-Ethylhexylisostearat verwendet.

In einer besonders bevorzugten Ausführungsform besteht die verwendete Ölkomponente ausschließlich aus dem Wachs Cetylpalmitat.

Weiterhin werden vorzugsweise 2-Ethylhexylisostearat, Octyldodecanol, Isotridecylisononanoat, Isoeicosan, 2-Ethylhexylcocoat, C₁₂₋₁₅- Alkylbenzoat, Capryl-Caprinsäuretriglycerid, Dicaprylylether und deren Mischungen als Ölkomponente verwendet.

Gegebenenfalls ist mindestens ein bakteriostatisches Mittel, Pflegestoff, Lichtschutzmittel. desodorierender Wirkstoff, Farbstoff, Pigment, Duftstoff, Geruchsabsorber, Dispergator, Enzym. Stabilisator, fungistatisches Mittel, Emulgator, Coemulgator, Weichmacher, Konservierungsstoff, Antioxidans und/oder Feuchthaltemittel als pharmazeutische und/oder pharmazeutische Hilfs-, Verarbeitungs- und/oder Zusatzstoffe in der erfindungsgemäßen pharmazeutischen Zusammensetzung enthalten, deren Gehalt zwischen 0 bis 20 Gew.-% liegt, basierend auf der Gesamtzusammensetzung.

Erfindungsgemäß werden insbesondere nicht-ionische Emulgatoren verwendet, die im Allgemeinen eine bessere Hautverträglichkeit als anionische Emulgatoren aufweisen. Vorzugsweise werden Emulgatoren verwendet, die Anlagerungsprodukte von 10 bis 50 Mol-% Ethylenoxid an Fettalkohole, Fettsäuren, Fettsäurealkanolamide, Fettsäuremonoglyceride, Sorbitanfettsäureester, Methylglycosidfettsäureester oder Polyglycolether-modifizierte Polysiloxane sind.

Der Zusatz eines Coemulgators ist vorteilhaft zur Ausbildung von sehr feinteiligen Dispersionen und weist im Allgemeinen einen lipophilen Charakter auf. Erfindungsgemäß verwendete Coemulgatoren umfassen gesättigte Alkohole mit 8 bis 24 Kohlenstoffatomen, ethoxylierte oder propoxylierte Alkohole und Carbonsäuren mit 8 bis 24 Kohlenstoffatomen, Ester aus einem Polyol und einer Fettsäure, Mono- und Polyglycerinester, Mono- und Polyglycerinether, Propylenglycolester, Methylglucoseester, Polyglycerinmethylglucoseester und deren Mischungen.

Um die Feuchtkeitsregulierung der Haut weiterhin zu unterstützen, kann die erfindungsgemäße Zusammensetzung weiterhin Feuchthaltemittel umfassen. Erfindungsgemäß werden insbesondere 1,2-Propandiol, 1,4-Butandiol, Polyole wie Glycerin, Diglycerin, Triglycerin, 1,2,6-Hexantriol, Ethylenglycol, Propylenglycol, Butylenglycol, Erythrit, Polyethylenglycol-4 (PEG-4), PEG-6, PEG-7, PEG-8, PEG-9, PEG-10, PEG-12, PEG-14, PEG-16, PEG-18, PEG-20, Sorbeth-6, Sorbeth-20, Sorbeth-30, Sorbeth-40, Fructose, Glucose, Maltose, Sorbit, Sucrose, Maltitol, Inosit, Xylose, Xylit, Glucoronsäure, Hyaluronsäure, Pyrrolidoncarbonsäure und deren Mischungen verwendet.

Beispiele der erfindungsgemäß verwendeten Konservierungsstoffe umfassen Benzoesäure und ihre Derivate, wie beispielsweise Ammonium-, Natrium-, Kalium-, Magnesium-, Propyl-, Butylund Phenylbenoatbenzoat, Salicylsäure, Phenol, Benzylalkohol, 4-Hydroxybenzoesäure, 4-Hydroxybenzoesäureester, Triclosan, Halocarban, Hydantoin, Harnstoff, Sorbinsäure Dehydroessigsäure, Isoxazol, Oxazol, quartemäre Ammoniumverbindungen, Formaldehyd, Glutaraldehyd, Glyoxal, Hexamidin, Isopropylkresol, Peptid-Antibiotika, die Salze und/oder Derivate der vorstehenden Verbindungen und deren Mischungen.

Jeder Duftstoff kann verwendet werden, soweit er üblicherweise in pharmazeutischen Zusammensetzungen verwendet wird.

Erfindungsgemäß verwendete Geruchsabsorber sind beispielsweise Zeolithe, Zinkricinoleat, Cyclodextrine, Metalloxide, Chlorophyll und Silikate. Als Metalloxid wird vorzugsweise Aluminiumoxid verwendet. Vorzugsweise verwendete Silikate sind ausgewählt aus der Gruppe der Schichtsilikate, insbesondere Kaolinit, Montmorillonit, Saponit, Hectorit, Bentonit, Talkum, Ilit, Smectit, Beidelit und Nontronit.

Als Pigmente werden erfindungsgemäß Titandioxid, Eisenoxid, Zinkoxid und Kaolin verwendet, um die pharmazeutische Akzeptanz des Präparats beim Anwender zu unterstützen.

Erfindungsgemäß werden unter desodorierenden Wirkstoffen Stoffe verstanden, die als Geruchsüberdecker, Geruchsabsorber und keimhemmende Mittel wirken. Keimhemmende Mittel umfassen beispielsweise Organohalogenide.

Als Antioxidantien können Aminosäuren, a-Hydroxysäuren, Peptide, Imidazol, Carotine, Carotinoide, Thioverbindungen, Sulfoximinverbindungen, Metallchelatoren, Folsäure, Bilirubin, Catechine, Flavonoide, Tocopherol, Gallussäureester, Hydrochinon, Ascorbinsäure, Isoascorbinsäure, Tocopherole, Zimtsäure, Butylhydroxytoluol, Butylhydroxyanisol, Harnsäure, Mannose, Zinkverbindungen, Selenverbindungen und die Derivate oder Salze der vorstehenden Verbindungen verwendet werden. Weiterhin können Antioxidantien-haltige Pflanzenextrakte eingesetzt werden.

Als Beispiele für Weichmacher können Di-n-butylphthalat, Diethylsebacat, Diisopropyladipat, Ethylethylcarbomethylphthalat, Tetradecan, Propylenethylenglycolmonolaurat, ethoxyliertes oder propxyliertes Butanol, Hexylenglycol und Ethyl-1,3-Hexandiol genannt werden.

Weiterhin können in der erfindungsgemäßen Zusammensetzung Aloe vera und/oder Konjac-Manane enthalten sein.

In einer bevorzugten Ausführungsform der Erfindung enthält die Zusammensetzung weiterhin Hydroxyalkylcellulosen wie beispielsweise Hydroxyethylcellulose, Hydroxypropylcellulose, Hydroxydecylcellulose, Methylethoxyhydroxylcellulose, Polyacrylsäure, Polyvinylpyrrolidon, Polydimethylsiloxan, Polymethylphenylsiloxan, Polyacrylat, Siliciumdioxid, Aluminiumsilikat, Dextran, Vitamin A, Vitamin B₂, Vitamin E, Magnesiumascorbinsäure-2-phosphat, Dialkylglycyrrhizinat und/oder Glutathion.

In einer besonders bevorzugten Ausführungsform der vorliegenden Erfindung wird bei ölig-alkoholischen Gelen Siliciumdioxid oder ein Aluminiumsilikat und bei wässerig-alkoholischen oder alkoholischen Gelen ein Polyacrylat zugesetzt.

Weiterhin können der pharmazeutischen Zusammensetzung dem Fachmann geläufige Lichtschutzmittel, Stabilisatoren, Dispergatoren, Farbstoffe, Enzyme, fungistatische Mittel, bakteriostatische Mittel, keimtötende Mittel und Pflegestoffe zugesetzt werden.

Eine besonders bevorzugte Ausführungsform der vorliegenden Erfindung umfasst eine pharmazeutische Zusammensetzung, die 5-20 Gew.-% Natriumchlorid, Glycerin, Sorbitol, Propylenglycol, Ethoxydiglycol, Aloe vera, Konjac-Manane, Hyaluronsäure, Squalan und einen pH-Wert zwischen 4,0 bis 7,0 aufweist.

Die pharmazeutische Zusammensetzung kann in Form von Gel, Lipogel, Creme, Salbe, Lotion, Schaum, mit und ohne Treibmittel oder Druckgas vorliegen. Im Sinne der Erfindung schließt dies Emulsionen, Oleogele, Hydrodispersionen und Lipodispersionen ein.

Als Treibmittel oder Druckgas können erfindungsgemäß entweder ein verflüssigbares Gas oder ein Permanentgas oder eine Mischung der vorstehenden verwendet werden. Beispiele für verflüssigbare gasförmige Treibmittel sind Fluorkohlenwasserstoffe und deren Mischungen und Kohlenwasserstofftreibmittel und deren Mischungen, wie beispielsweise Butan, Isobutan, Propan oder Isopentan. Als Permanentgase sind Kohlendioxid, Stickstoff oder Distickstoffmonoxid geeignet. Die Menge des verwendeten Treibmittels wird dabei durch die üblichen, auf dem Aerosolgebiet bekannten Faktoren bestimmt. Weiterhin kann die pharmazeutische Zusammensetzung gemäß der Erfindung auch in Form eines Sprays ohne Verwendung eines Treibmittel abgegeben werden, wenn eine sie in einem mit einer fingerbetriebenen Pumpe versehenen Behälter enthalten ist.

Die pharmazeutische Zusammensetzung gemäß der vorliegenden Erfindung wird zur Herstellung eines topischen Präparats zur Prophylaxe und/oder Behandlung von Hautekzemen, -rötungen, -trockenheit, -einrissen, -schuppungen und -juckreiz verwendet. In besonders bevorzugten Ausführungsformen wird die Zusammensetzung zur Herstellung eines topischen Präparats zur Prophylaxe von Hautekzemen, -rötungen, -trockenheit, -einrissen, -schuppungen und juckreiz eingesetzt. Das topische Präparat kann allgemein bei Säugetieren eingesetzt werden. Eine bevorzugte Zielgruppe sind Menschen, das topische Präparat kann aber auch bei Tieren angewendet werden.

Folgende Beispiele und Figuren sollen die Erfindung erläutern, ohne sie jedoch einzuschränken.
Figur 1 zeigt die Wirkung der Behandlung mit erfindungsgemäßen und nicht erfindungsgemäßen Zusammensetzungen nach Okklusion durch Beurteilung mittels Episkopie im Vergleich zu Kontrollen.
Fig. 2 zeigt die Wirkung der Behandlung mit erfindungsgemäßen und nicht erfindungsgemäßen Zusammensetzungen nach Okklusion durch Beurteilung mittels eines Comeometers im Vergleich zu Kontrollen.
Fig. 3 zeigt die Wirkung der Behandlung mit erfindungsgemäßen und nicht erfindungsgemäßen Zusammensetzungen nach Okklusion durch Beurteilung mittels eines Tewameters im Vergleich zu Kontrollen.
Figur 4 zeigt eine erste Kammerpenetrationen der Kontrolle.
Figur 5 zeigt eine erste Kammerpenetrationen unter Verwendung von NaOH.
Figur 6 zeigt eine erste Kammerpenetration einer erfindungsgemäßen Zusammensetzung.
Figur 7 zeigt erste Kammerpenetration einer nicht erfindungsgemäßen Zusammensetzung.
Figuren 8, 9, 10 bzw. 11 zeigen eine zweite Kammerpenetration der Kontrolle, unter Verwendung von NaOH, einer erfindungsgemäßen Zusammensetzung bzw. nicht erfindungsgemäßen Zusammensetzung.
Figuren 12, 13, 14 bzw. 15 zeigen eine dritte Kammerpenetration der Kontrolle, unter Verwendung von NaOH, einer erfindungsgemäßen Zusammensetzung bzw. nicht erfindungsgemäßen Zusammensetzung.
Figuren 16, 17, 18 bzw. 19 zeigen eine vierte Kammerpenetration der Kontrolle, unter Verwendung von NaOH, einer erfindungsgemäßen Zusammensetzung bzw. nicht erfindungsgemäßen Zusammensetzung.
Figuren 20, 21, 22 bzw. 23 zeigen eine fünfte Kammerpenetration der Kontrolle, unter Verwendung von NaOH, einer erfindungsgemäßen Zusammensetzung bzw. nicht erfindungsgemäßen Zusammensetzung.

Die Erfindung wird nun durch Beispiele erläutert, die jedoch nur veranschaulichend sind und den Schutzumfang der Erfindung nicht einschränken.

| **Beispiel 1** | | |
|---|---|---|
| Fitoderm | 8 Gew.-% | |
| Emulgade CM | 5 Gew.-% | |
| Konjac Mannane 1,0 MEP | 2,5 Gew.-% | |
| Natrosol 250 HHBR | 0,5 Gew.-% | |
| Natriumchlorid | 10 Gew.-% | |
| Natronlauge 45% | 0,2 Gew.-% | Parfümöle, Wasser ad 100 Gew.-% |
| | | pH-Wert 5,5 |

| **Beispiel 2** | | |
|---|---|---|
| PEG-40 hydriertes | | |
| Rizinusöl | 2 Gew.-% | |
| Glycerin | 2 Gew.-% | |
| Natriumchlorid | 10 Gew.-% | |
| Milchsäure | 1,6 Gew.-% | |
| Na-Lactat | 15 Gew.-% | Parfümöle, Wasser ad 100 Gew.-% |
| | | pH-Wert 5,5 |

| **Beispiel 3** | | |
|---|---|---|
| Natriumchlorid | 10 Gew.-% | |
| FinsolvTN | 3,25 Gew.-% | |
| MC 30 | 3,25 Gew.-% | |
| Montanov 68 | 3 Gew.-% | |
| Bienenwachs, weiß | 1,5 Gew.-% | |
| Lactolin pH 5,6 | 1 Gew.-% | |
| Keltrol TF | 0,3 Gew.-% | |
| Natronlauge | 0,1 Gew.-% | Parfümöle, Wasser ad 100 Gew.-% |
| | | pH-Wert 5,5 |

| **Beispiel 4** | | |
|---|---|---|
| Octyldodecanol | 2 Gew.-% | |
| C₁₂₋₁₅Alkylbenzoat | 2 Gew.-% | |
| C₁₀₋₁₀Alkylacrylat | 0,15 Gew.-% | |
| Natriumchlorid | 10 Gew.-% | |
| Milchsäure | 1,6 Gew.-% | |
| Na-Lactat | 15 Gew.-% | Parfümöle, Wasser ad 100 Gew.-% |
| | | pH-Wert 5,5 |

| **Beispiel 5** | | |
|---|---|---|
| Xanthan Gum | 1 Gew.-% | |
| Milchsäure | 1,6 Gew.-% | |
| Sorbit | 3 Gew.-% | |
| Propylenglycol | 3 Gew.-% | |
| Glycerin | 3 Gew.-% | |
| Natriumchlorid | 10 Gew.-% | |
| Na-Lactat | 15 Gew.-% | Parfümöle, Wasser ad 100 Gew.-% |
| | | pH-Wert 5,5 |

| **Beispiel 6** | | |
|---|---|---|
| Natriumchlorid | 10 Gew.-% | |
| Milchsäure | 1,6 Gew.-% | |
| Na-Lactat | 15 Gew.-% | |
| Hydroxydecylcellulose | 1 Gew.-% | |
| Parfüm | 1 Gew.-% | Parfümöle, Wasser ad 100 Gew.-% |
| | | pH-Wert 5,5 |

| **Vergleichsbeispiel 1 (VB1)** | | |
|---|---|---|
| Locron LIC Fl. | 21 Gew.-% | |
| Lactolin pH 5,6 | 15 Gew.-% | |
| Natrural Extract AP | 5 Gew.-% | |
| Chloracel fest | 4,4 Gew.-% | |
| Dehyquart A-CA | 3 Gew.-% | |
| Purac 80% | 2,1 Gew.-% | |
| Merquat 281 | 2 Gew.-% | Parfumöle, Wasser ad 100 Gew.-% |
| | | pH-Wert 5,5 |

| **Vergleichsbeispiel 2 (Vergleichsbeispiel)** | | |
|---|---|---|
| Locron L | 21 Gew.-% | |
| Butylen Glycol | 5 Gew.-% | |
| Crodamol PMP | 5 Gew.-% | |
| Emulgade CM | 5 Gew.-% | |
| Natural Extract AP | 5 Gew.-% | |
| Chloracel fest | 4,4 Gew.-% | |
| Natronlauge 45% | 1 Gew.-% | |
| Structure Solanace | 0,9 Gew.-% | |
| Zitronensäure-Monohydrat | 0,8 Gew.-% | |
| Natrosol 250 HBR | 0,45 Gew.-% | Parfümöle, Wasser ad 100 Gew.-% pH-Wert 5,5 |

Vorstehende Ausgangsstoffe haben folgende INCI-Namen und können von dem angegeben Hersteller bezogen werden.

| | |
|---|---|
| Fitoderm: | INCI: Squalan, Hersteller: Cognis |
| Emulgade CM | INCI: Cetearylisononanoat (und) Ceterareth-20 (und) Cetearylalkohol (und) Glycerylstearat (und) Glycerin (und) Cetylplarnitat (und) Ceteareth-12, Hersteller: Henkel |
| Konjac Mannane: | INCI: Mannan, Hersteller: GfN |
| Natrosol 250 HHBR: | INCI: Hydroxyethylcellulose, Hersteller: Hercules |
| FinsolvTN: | INCI: C12-C15 Alkylbenzoat, Hersteller: Erbslöh |
| MC 30: | INCI: hydriertes Polyisobuten, Hersteller: N&R |
| Montanov 68: | INCI: Cetearylalkohol und Cetearylglucosid, Hersteller: Interorgana |
| Bienenwachs, weiß: | INCI: Cera alba, Hersteller: Kahl & Co. |
| Lactolin: | INCI: Natriumlactat (50%ige Lösung), Hersteller: Boehringer |
| Keltrol TF: | INCI: Xanthan Gum (94%ige Lösung), Hersteller: Biesterfeld |
| Chloracel fest: | INCI: Natriumaluminiumchlorhydroxylactat, Hersteller: Interorgana |
| Locron L: | INCI: Aluminiumchlorohydrat (50%ige Lösung), Hersteller: Höchst AG |
| Locron LIC Fl: | INCI: Aluminiumchlorohydrat (50%ige Lösung), Hersteller: Clariant |
| Dehyquart A-CA: | INCI: Cetrimoniumchlorid (30%ige Lösung), Hersteller: Cognis |
| Purac: | INCI: Natriumlactat (80%ige Lösung), Hersteller: Purac Biochem |
| Merquat 281: | INCI: Polyquaternium-22 (42,5%ige Lösung), Hersteller: Chemviron |
| Crodamol PMP: | INCI: PPG-2-Myristyletherpropionat, Hersteller: Croda GmbH |
| Structure Solanace: | INCI: Modifizierte Kartoffelstärke (86%ige Lösung), Hersteller: S. Black |
| Natrosol 250 HBR: | INCI: Hydroxyethylcellulose, Hersteller: Hercules |
| Natural Extract AP: | INCI: Betain, Hersteller: Rovi GmbH |

Um die Wirksamkeit der vorliegenden Zusammensetzungen darzulegen, wurde der Einfluss zweier erfindungsgemäßer Zusammensetzungen gemäß Beispiel 1 (B1) und Beispiel 3 (B3) auf experimentell gequollene Homschicht in *vivo* und *in vitro* untersucht. Die Untersuchungen wurden unter Verwendung von dem Fachmann bekannten Geräten, Messmethoden und Messbedingungen durchgeführt, wenn nicht anders angegeben.

### Untersuchung zum Einfluss zweier erfindungsgemäßer Zusammensetzungen auf experimentell gequollene Hornschicht in vivo

Das methodische Vorgehen simulierte eine Situation, wie sie beim prolongierten Tragen okklusiv wirkender Operationshandschuhe nach vorheriger chirurgischer Handwäsche gegeben ist. Dazu wurde entsprechend des Vorgehens beim repetitiven Waschtest die Unterarmbeugeseite von 15 freiwilligen Probanden mit NLS gewaschen (Gehring et al, Predictive Washing Test for Evaluation the Individual Eczema Risk. Contact Dermatitis 39, 8-13. 1998). Im Anschluss an die Waschung wurde das gesamte Testfeld mit einer Folie für 24 Stunden okklusiv abgedeckt. Unmittelbar nach der Okklusionsphase wurden die zwei vorstehenden Zusammensetzungen standardisiert appliziert und deren Soforteffekt nach 5 Minuten beurteilt.

Die Okklusion führt aufgrund eines Quelleffektes zu einer Vergröberung des Hautoberflächenreliefs und zu einer ausgeprägteren Hautrauhigkeit. Damit ist ein Anstieg des transepidermalen Wasserverlustes verbunden, der die okklusiv bedingte Irritation widerspiegelt. Unmittelbar nach Okklusion geht der erhöhte transepidermale Wasserverlust in die Kapazitätsmessung mit ein, so dass sich erhöhte Werte darstellen. Bereits 5 Minuten später wird allerdings - ebenfalls als Zeichen der Irritation - ein Verlust an Hornschichtfeuchtigkeit ersichtlich.

Die Hautrauhigkeit wurde episkopisch mit dem Visiometer beurteilt (Ruhr J.W., Gehring W., Gloor M.: Analyse der Hautrauhigkeit bei Personen unterschiedlicher Altersgruppen mit dem Visiometer - Ein neuartiges EDV-gestütztes Messverfahren. Akt. Denn. 21, 151-156, 1995). Bei diesem Verfahren drückt eine Abnahme des Messwertes eine Hautglättung und eine Verminderung der Hautrauhigkeit aus.

Die Untersuchungen erfolgten an den Vorlarseiten beider Unterarme. Die Zusammensetzung gemäß Beispiel 1 wurde zusammen mit einem unbehandelten Kontrollfeld (Kontrolle 1, Kl) zu einer Gruppe zusammengefasst und an einem Unterarm getestet. Kontralateral erfolgte die Untersuchung der Zusammensetzung gemäß Beispiel 3 im Vergleich zu einem unbehandelten Kontrollfeld (Kontrolle 2, K2). Alle Messwerte sind Differenzen zum Ausgangswert nach Okklusion (AW). Die statistische Auswertung erfolgte für jeden Testblock getrennt nach dem Wilcoxon Paardifferenzentest für verbundene Stichproben.

Die Untersuchungen erfolgten an einem Kollektiv von 15 gesunden Probanden. Davon waren 11 weiblich mit einem Durchschnittsalter von 36,5 Jahren. Die Altersgrenzen lagen bei 20 und 60 Jahren. Die 4 männlichen Probanden hatten ein Durchschnittsalter von 32,5 Jahren. Hier waren Altersgrenzen von 19 und 48 Jahren gegeben.

### Die Ergebnisse sind wie folgt:

### Episkopie

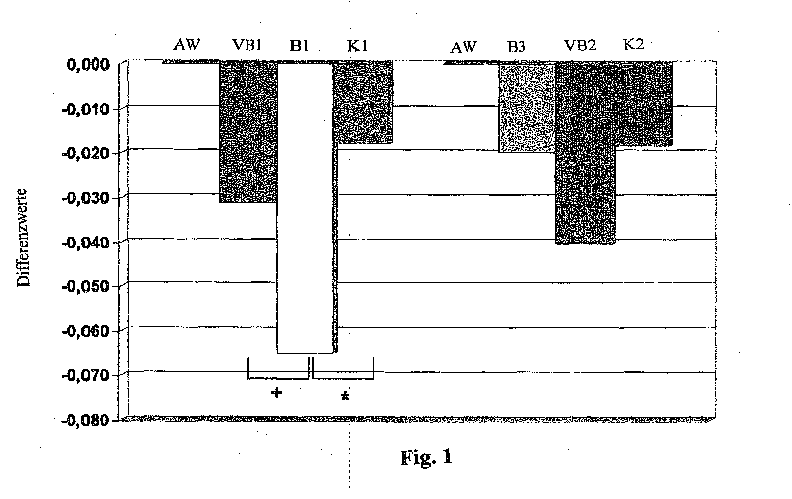

Nach Beendigung der Okklusion kommt es auch unbehandelt zu einer Reduktion der Hautrauhigkeit, die allerdings insbesondere durch Anwendung von der Zusammensetzung gemäß Beispiel 1 verbessert wird. Beispiel 1 hebt sich statistisch signifikant von dem unbehandelten Kontrollfeld ab. Die Ergebnisse sind Fig. 1 dargestellt. Fig. 1 zeigt die Wirkung der Behandlung mit den erfmdungsgemäßen Zusammensetzungen nach Okklusion durch Beurteilung mittels Episkopie im Vergleich zu Kontrollen.

### Transepidermaler Wasserverlust

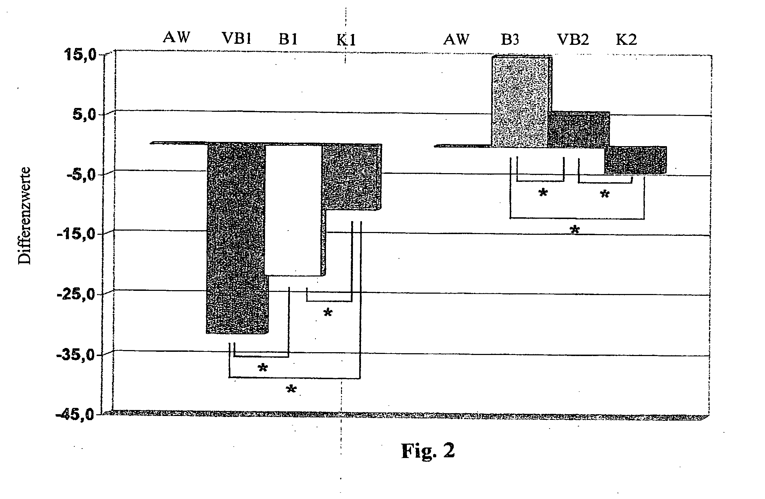

Die Zusammensetzung gemäß Beispiel 1 hat eine bessere Reduktion des irritativ bedingten Anstiegs des TEWL herbeigeführt als dies bei der unbehandelten Kontrolle der Fall gewesen ist. Die Ergebnisse sind in Fig. 2 dargestellt. Fig. 2 zeigt die Wirkung der Behandlung mit den erfindungsgemäßen Zusammensetzungen nach Okklusion durch Beurteilung mittels eines Corneometers im Vergleich zu Kontrollen.

### Hornschichtfeuchtigkeit

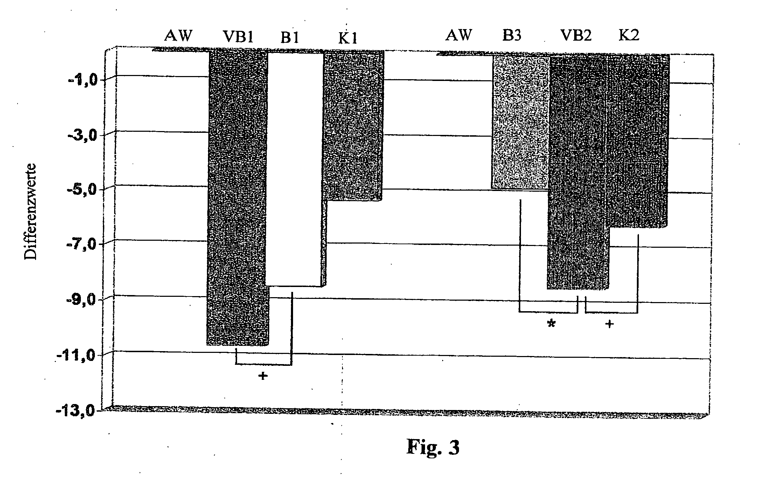

Insbesondere die Zusammensetzung gemäß Beispiel 3 hat den durch die Okklusion bedingten Verlust an Hornschichtfeuchtigkeit vollständig verhindert und im Sinn einer Positivbilanz gegenüber zum unbehandelten Kontrollfeld zu einer statistisch signifikanten Verbesserung der Hornschichtfeuchtigkeit geführt. Die Ergebnisse sind in Fig. 3 dargestellt. Fig. 3 zeigt die Wirkung der Behandlung mit den erfindungsgemäßen Zusammensetzungen nach Okklusion durch Beurteilung mittels eines Tewameters im Vergleich zu Kontrollen.

Prolongierte Okklusion in Kombination mit intensiver Hautreinigung durch NLS führt zu einer Zunahme der Hautrauhigkeit, zu einem erhöhten transepidermalen Wasserverlust und zu einer Reduktion der Hornschichtfeuchtigkeit. Eine strikte Korrelation des Effektes der zwei verschiedenen Zusammensetzung bei der Beeinflussung der unterschiedlichen Bewertungskriterien (Hautrauhigkeit, TEWL, Hornschichtfeuchtigkeit) ist nicht gegeben. Insbesondere Beispiel 1 zeigt einen hervorragenden glättenden Einfluss auf die Hautoberfläche.

### Untersuchungen zum Einfluss zweier erfindungsgemäßer Zusammensetzungen auf experimentell gequollene Hornschicht in vitro

Bei einer vorausgegangenen Untersuchung wurden zwei Zusammensetzungen gemäß Beispiel 1 bzw. Beispiel 3 auf ihren hautglättenden Einfluss bei experimentell durch Okklusion gequollener Hornschicht untersucht. Dabei ließ die Zusammensetzung gemäß Beispiel 1 episkopisch betrachtet die größte Abnahme der Hautrauhigkeit erkennen. Ziel der weiterführenden Untersuchung war es, den direkten Einfluss auf das gequollenen Stratum corneum zu visualisieren.

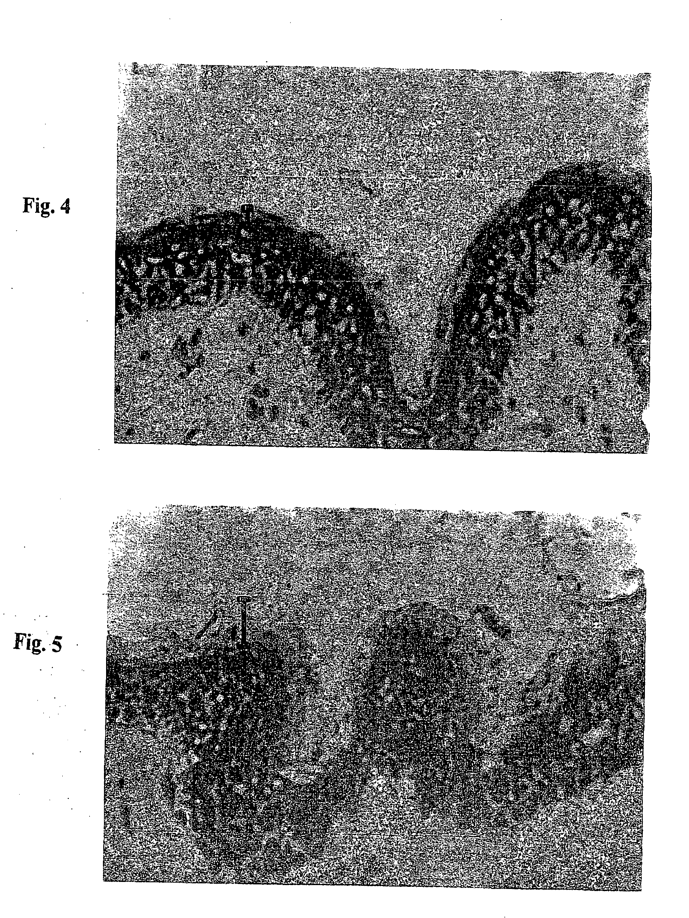

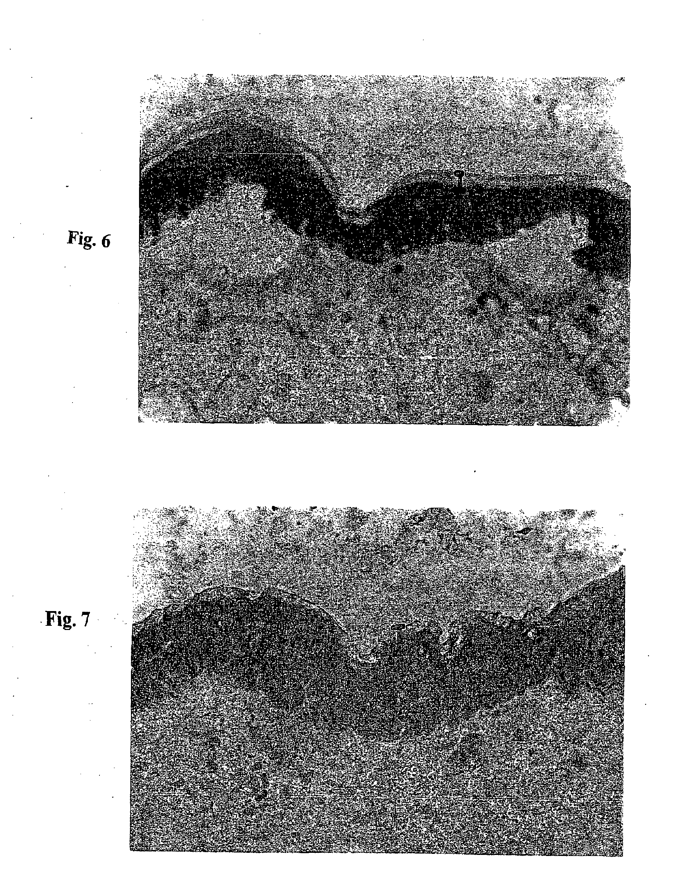

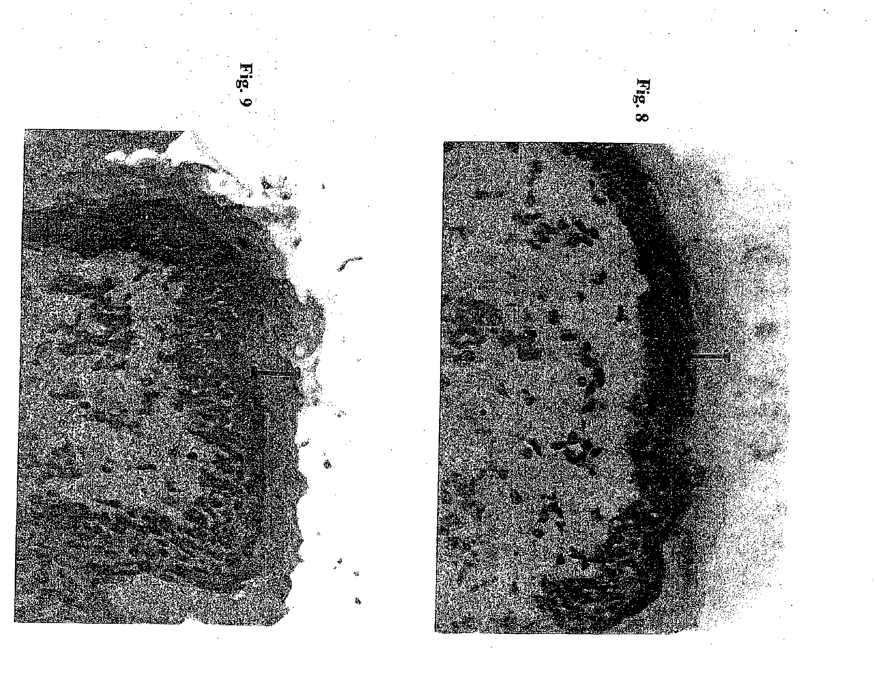

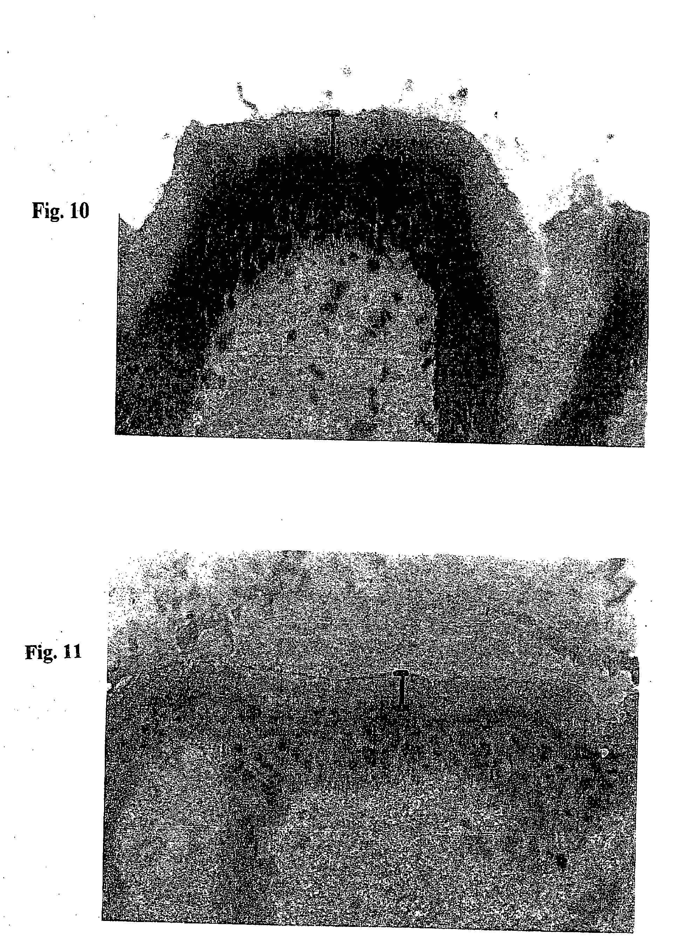

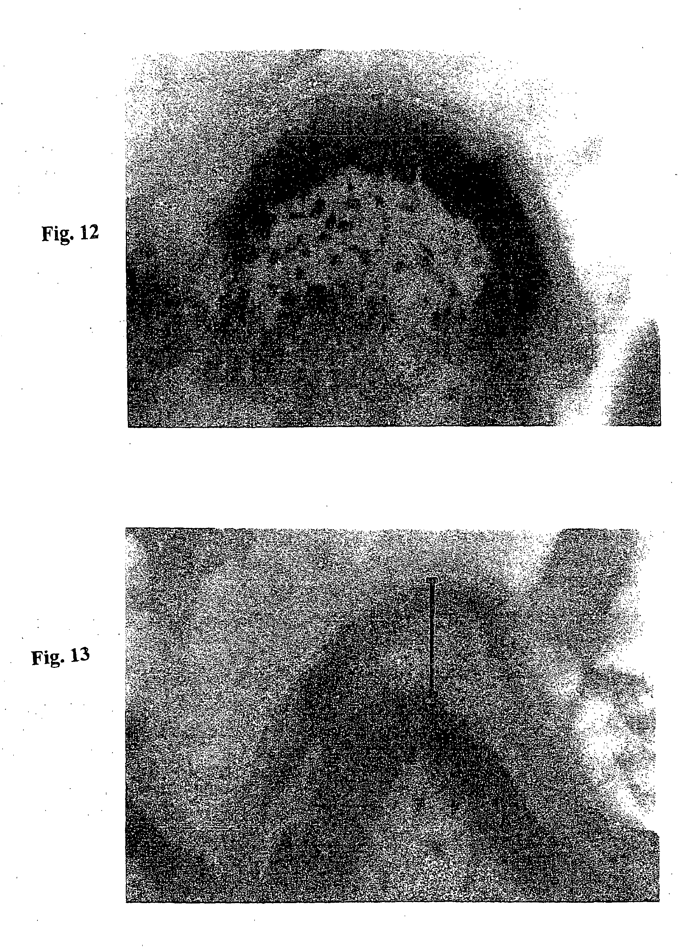

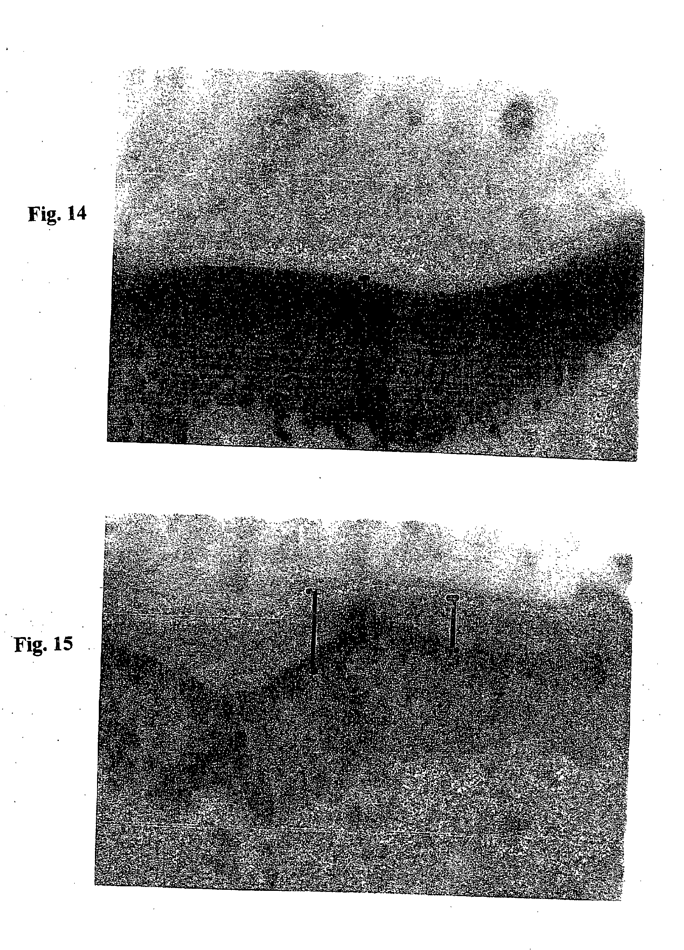

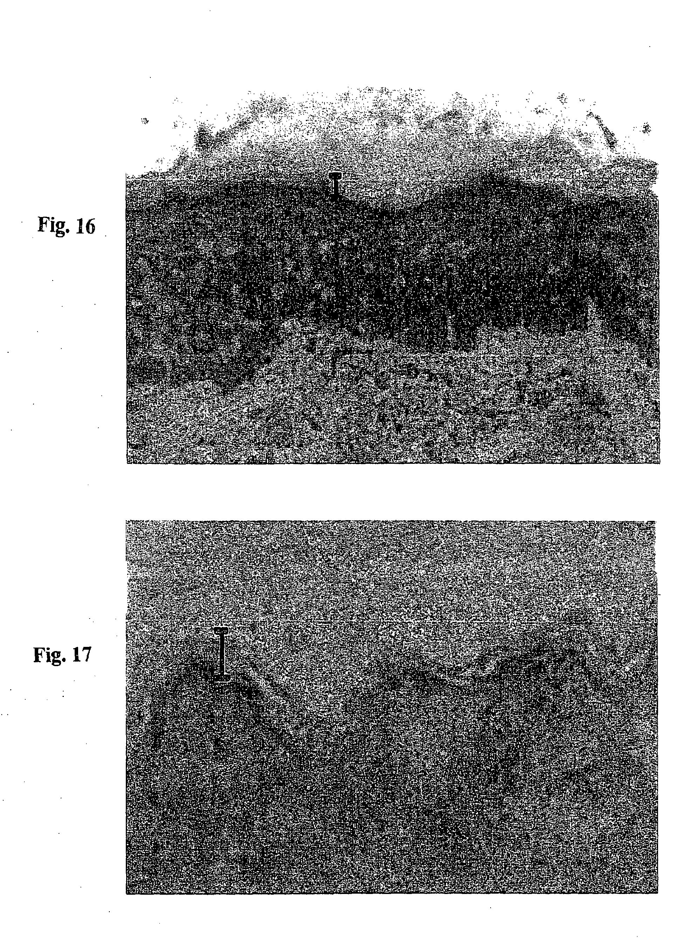

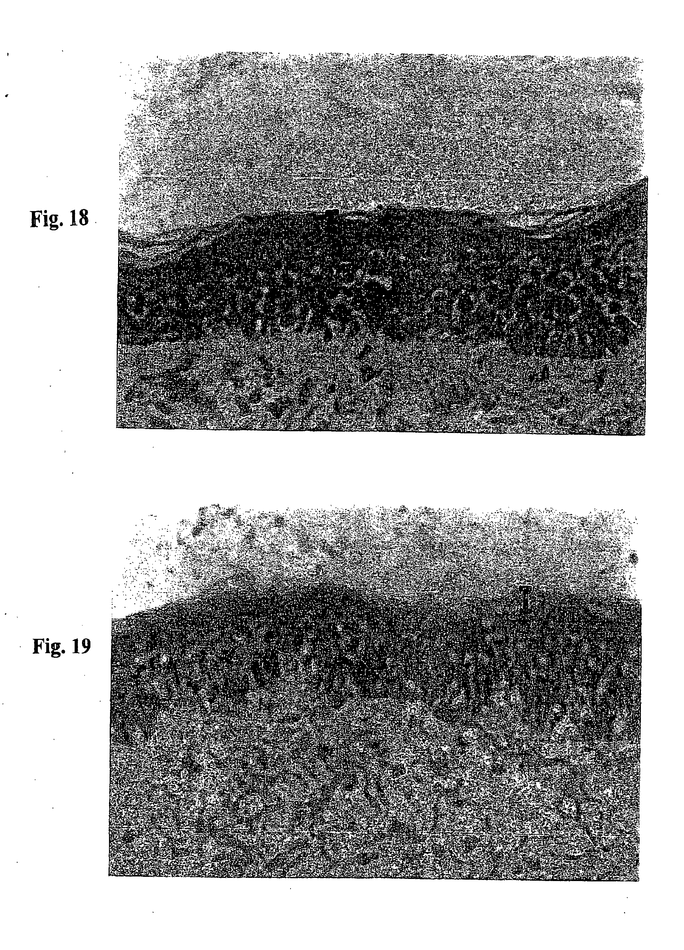

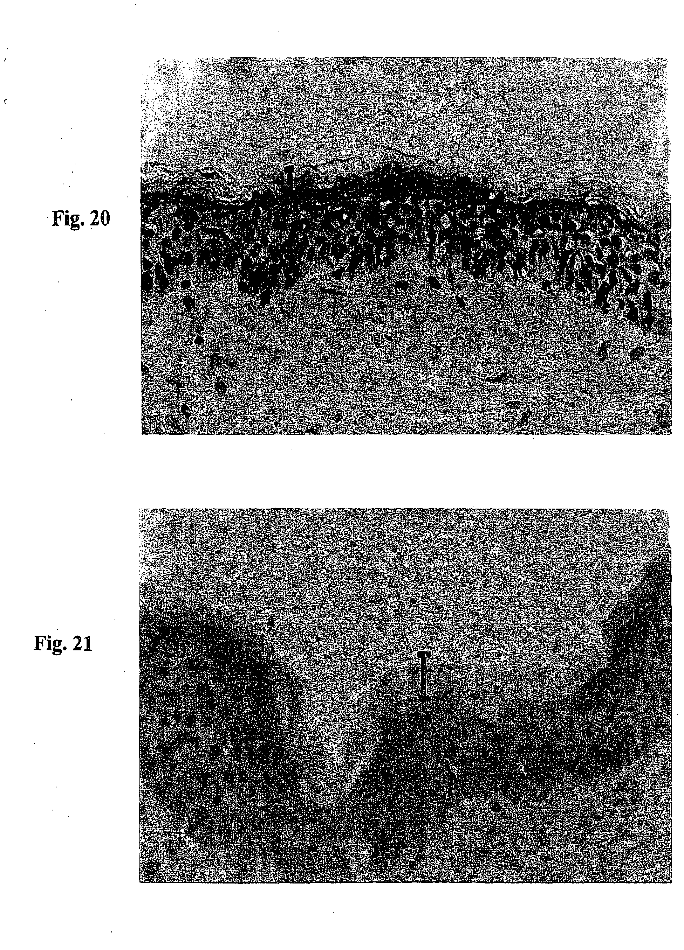

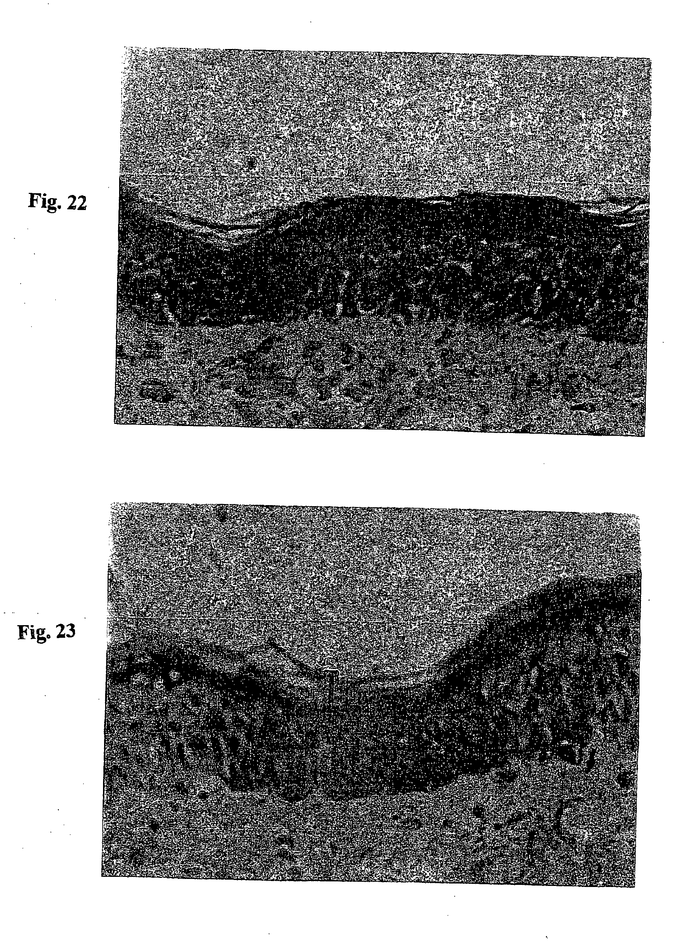

Bei der Zusammensetzung gemäß Beispiel 1 handelt es sich um eine klare, transparente Formulierung, die am Gefrierschnittmodell beurteilt werden konnten, wobei im vorliegenden Fall zehn Gefrierschnittmodelle beurteilt wurden. Für die Zusammensetzung cremiger, nicht transparenter Konsistenz - wie dies bei der Zusammensetzung gemäß Beispiel 3 der Fall ist -verbietet sich dieses Verfahren. Hier wurde eine modifizierte Kammerpenetration in zwei Schritten vorgenommen. Zuerst erfolgte eine Inkubation mit NaOH, um eine Hornschichtquellung zu provozieren. Nach 5 Minuten Inkubationszeit wurden Überstände von NaOH abpippetiert. Nach anschließender Spülung mit NaCI erfolgte die Behandlung mit der Zusammensetzung gemäß Beispiel 3. Abschließend wurde unbehandelte, mit NaOH gequollene und mit der Zusammensetzung inkubierte Haut mikroskopisch untersucht. Die histologischen Präparate wurden nach HE gefärbt. Die Ergebnisse der fünf durchgeführten Kammerpenetrationen der Zusammensetzung gemäß Beispiel 3, gemäß Vergleichsbeispiel 2 sowie der Kontrolle und mit Natronlauge sind in Figur 4 bis 23 dargestellt.

Bei der Untersuchung am Kryostatschnitt hat die Zusammensetzung gemäß Beispiel 1 bei allen 10 untersuchten Gewebeproben übereinstimmend eine hervorragende entquellende Wirkung auf das experimentell gequollene Stratum corneum erkennen lassen. Bei der Kammerpenetration hat die Zusammensetzung gemäß Beispiel 3 ebenfalls sehr gute Resultate gezeigt. Bei Gewebe Nr. 2 ist die unbehandelte Homschicht schon erheblich gequollen.

Mit beiden Vorgehensweisen, d. h. Gefrierschnitt und Kammerpenetration, ist die morphologische Beurteilung der entquellenden Wirkung an der Homschicht möglich gewesen. Die Ergebnisse sind eindeutig und gut reproduzierbar und zeigen deutliche die Wirkung der erfindungsgemäßen Zusammensetzungen.

### LITERATUR:

[1]
   Hornstein: "Berufsbedingte allergische Dermatosen", TW Dermatologie 25, 110 - 121, 1995
[2]
   Göring, Kröning, Ziemer: "Kontaktallergien bei medizinischem Personal", Der Deutsche Dermatologe 7, 727 - 733, 1993
[3]
   Fluhr, Lazzerini, DIstante, Gloor, Berardesca: "Effects of Prolonged Occlusion on Stratum corneum Barrier Function and Water Holding Capacity", Skin Pharmacol Appl Skin Physiol 1999, 12: 193 - 198
[4]
   Odia, Pürschel, Vocks, Rakoski: "Noxen und Irritantien im Beruf", Dermatosen 42, Heft 5, 179 - 183, 1994
[5]
   Schwanitz: "Prävention chronischer Handekzeme", Der Deutsche Dermatologe 9, 931 - 938, 1993
[6]
   Pilz, Frosch: "Hautschutz für Friseure", Dermatosen 42, Heft 5, 199 - 202, 1994
[7]
   Gehring: "Desikierende Wirkung von Emulgatoren" (Nachweis über Autor)
[8]
   Gloor: "Pharmakologie dermatologischer Extema", Springer-Verlag, S. 84 - 85, 1982.
[9]
   Korting, Sterry: "Therapeutische Verfahren in der Dermatologie, Dermatika und Kosmetika", Blackwell Wissenschaftsverlag 2001, S. 175.

## Patentansprüche

1. Pharmazeutische Zusammensetzung mit hautentquellender Wirkung, die aus mindestens einem Alkali- und/oder Erdalkalichlorid als Wirkstoff und einem pharmazeutisch akzeptablen Träger als Grundlage besteht, der ausgewählt ist aus der Gruppe, bestehend aus einem flüssigen Basismaterial, einem pH-Regulator, und gegebenenfalls einer Ölkomponente.

2. Pharmazeutische Zusammensetzung mit hautentquellender Wirkung Anspruch 1, **dadurch gekennzeichnet, dass** das mindestens eine Alkali- und/oder Erdalkalichlorid ausgewählt ist aus der Gruppe, bestehend aus Natriumchlorid, Calciumchlorid, Magnesiumchlorid, Kaliumchlorid und deren Mischungen, wobei Natriumchlorid besonders bevorzugt ist.

3. Pharmazeutische Zusammensetzung mit hautentquellender Wirkung gemäß Anspruch 1 oder 2, **dadurch gekennzeichnet, dass** die Zusammensetzung Natriumchlorid in einer Konzentration von 5 bis 20 Gew.-% aufweist.

4. Pharmazeutische Zusammensetzung mit hautentquellender Wirkung gemäß irgendeinem der vorangehenden Ansprüche 1 bis 3, **dadurch gekennzeichnet, dass** die Zusammensetzung einen pH-Wert zwischen 4,0 bis 7,0 aufweist.

5. Pharmazeutische Zusammensetzung mit hautentquellender Wirkung gemäß irgendeinem der vorangehenden Ansprüche 1 bis 4, **dadurch gekennzeichnet, dass** der pH-Wert der Zusammensetzung 5,5 ist.

6. Pharmazeutische Zusammensetzung mit hautentquellender Wirkung gemäß irgendeinem der vorangehenden Ansprüche 1 bis 5, **dadurch gekennzeichnet, dass** das flüssige Basismaterial Wasser, Alkohole mit 2 bis 4 Kohlenstoffatomen oder deren Mischungen umfasst.

7. Pharmazeutische Zusammensetzung mit hautentquellender Wirkung gemäß irgendeinem der vorangehenden Ansprüche 1 bis 6, **dadurch gekennzeichnet, dass** der pH-Regulator Milchsäure, Zitronensäure, Milchsäuresalze, Zitronensäuresalze oder deren Mischungen, wobei die Salze insbesondere Alkali- und Erdalkalisalze umfassen, oder Alkali umfasst.

8. Pharmazeutische Zusammensetzung mit hautentquellender Wirkung gemäß irgendeinem der vorangehenden Ansprüche 1 bis 7, **dadurch gekennzeichnet, dass** die Ölkomponente Ester aus gesättigten und/oder ungesättigten, verzweigten und/oder unverzweigten Alkoholen einer Kettenlänge von 3 bis 30 Kohlenstoffatomen und gesättigten und/oder ungesättigten, verzweigten und/oder unverzweigten Alkancarbonsäuren einer Kettenlänge von 3 bis 30 Kohlenstoffatomen und/oder aromatischen Carbonsäuren, Kohlenwasserstoffe, Kohlenwasserstoffwachse, Silikonöle, Dialkylether, gesättigte und/oder ungesättigte, verzweigte und/oder unverzweigte Alkohole und/oder Fettsäuretriglyceride oder deren Mischungen umfasst.

9. Pharmazeutische Zusammensetzung mit hautentquellender Wirkung gemäß Anspruch 8, **dadurch gekennzeichnet, dass** die Ölkomponente ausgewählt ist aus der Gruppe der Esteröle, bestehend aus Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleylerucat, Erucyloleat, Erucylerucat sowie synthetische, halbsynthetische und natürliche Gemische solcher Ester, aus der Gruppe der Triglycerinester gesättigter und/oder ungesättigter, verzweigter und/oder unverzweigter Alkancarbonsäuren einer Kettenlänge von 8 bis 24, vorzugsweise 12 bis 18 Kohlenstoffatomen, aus der Gruppe der synthetischen, halbsynthetischen und natürlichen Ölen, bestehend aus Olivenöl, Sonnenblumenöl, Sojaöl, Erdnussöl, Rapsöl, Mandelöl, Palmöl, Kokosöl, Jojobaöl, Nerzöl und Rizinusöl, aus der Gruppe der Kohlenwasserstoffe und -wachse, bestehend aus Paraffinöl, Squalan und Squalen, und/oder aus der Gruppe der Silikonöle, bestehend aus Octamethylcyclotetrasiloxan, Hexamethylcyclotrisiloxan, Polydimethylsiloxan, Poly(methylphenylsiloxan) und deren Mischungen.

10. Pharmazeutische Zusammensetzung mit hautentquellender Wirkung gemäß Anspruch 8, **dadurch gekennzeichnet, dass** die Ölkomponente Cetylpalmitat ist.

11. Pharmazeutische Zusammensetzung mit hautentquellender Wirkung gemäß Anspruch 8, **dadurch gekennzeichnet, dass** die Ölkomponente ausgewählt ist aus der Gruppe, bestehend aus 2-Ethylhexylisostearat, Octyldodecanol, Isotridecylisononanoat, Isoeicosan, 2-Ethylhexylcocoat, C₁₂₋₁₅- Alkylbenzoat, Capryl-Caprinsäuretriglycerid, Dicaprylylether und deren Mischungen.

12. Pharmazeutische Zusammensetzung mit hautentquellender Wirkung gemäß Anspruch 8, **dadurch gekennzeichnet, dass** die Ölkomponente ausgewählt ist aus der Gruppe, bestehend aus Mischungen aus C₁₂₋₁₅- Alkylbenzoat und 2-Ethylhexylisostearat, Mischungen aus C₁₂₋₁₅-Alkylbenzoat und Isotridecylisononanoat und Mischungen aus C₁₂₋₁₅-Alkylbenzoat, 2-Ethylhexylisostearat und Isotridecylisononanoat.

13. Pharmazeutische Zusammensetzung mit hautentquellender Wirkung gemäß Anspruch 8, **dadurch gekennzeichnet, dass** die Ölkomponente eine Mischung aus Octamethylcyclotetrasiloxan und Isotridecylisononanoat oder Mischungen aus Octamethylcyclotetrasiloxan und 2-Ethylhexylisostearat umfasst.

14. Pharmazeutische Zusammensetzung mit hautentquellender Wirkung gemäß irgendeinem der vorangehenden Ansprüche 1 bis 13, **dadurch gekennzeichnet, dass** die Zusammensetzung weiterhin mindestens einen üblichen pharmazeutischen und/oder pharmazeutischen Hilfs-, Verarbeitungs- und/oder Zusatzstoff aufweist, der ausgewählt ist aus der Gruppe, bestehend aus bakteriostatischen Mitteln, Pflegestoffen, Lichtschutzmitteln, desodorierenden Wirkstoffen, Farbstoffen, Pigrnenten, Duftstoffen, Geruchsabsorbern, Dispergatoren, Enzymen, Stabilisatoren und fungistatischen Mitteln.

15. Pharmazeutische Zusammensetzung mit hautentquellender Wirkung gemäß irgendeinem der vorangehenden Ansprüche 1 bis 14, **dadurch gekennzeichnet, dass** die Zusammensetzung weiterhin mindestens einen üblichen pharmazeutischen und/oder pharmazeutischen Hilfs-, Verarbeitungs- und/oder Zusatzstoff aufweist, der ausgewählt ist aus der Gruppe, bestehend aus Emulgatoren, Coemulgatoren, Weichmachern, Konservierungsstoffen, Antioxidantien, und Feuchthaltemitteln.

16. Pharmazeutische Zusammensetzung mit hautentquellender Wirkung gemäß irgendeinem der vorangehenden Ansprüche 1 bis 15, **dadurch gekennzeichnet, dass** die Zusammensetzung weiterhin mindestens einen üblichen pharmazeutischen und/oder pharmazeutischen Hilfs-, Verarbeitungs- und/oder Zusatzstoff aufweist, der ausgewählt ist aus der Gruppe, bestehend aus Hydroxyethylcellulose, Hydroxypropylcellulose, Hydroxydecylcellulose, Methylethoxyhydroxylcellulose, Polyacrylsäure, Polyvinylpyrrolidon, Polydimethylsiloxan, Polymethylphenylsiloxan, Polyacrylat, Siliciumdioxid, Aluminiumsilikat, Dextran, Vitamin A, Vitamin B₂, Vitamin E, Magnesiumascorbinsäure-2-phosphat, Dialkylglycyrrhizinat, Glutathion, Aloe vera und Konjac-Manane.

17. Pharmazeutische Zusammensetzung mit hautentquellender Wirkung gemäß Anspruch 16, **dadurch gekennzeichnet, dass** der mindestens eine übliche pharmazeutische und/oder pharmazeutische Hilfs-, Verarbeitungs- und/oder Zusatzstoff bei ölig-alkoholischen Gelen Siliciumdioxid oder ein Aluminiumsilikat ist und bei wässerig-alkoholischen oder alkoholischen Gelen ein Polyacrylat ist.

18. Pharmazeutische Zusammensetzung mit hautentquellender Wirkung gemäß Anspruch 16, die 5 bis 20 Gew.-% Natriumchlorid, Glycerin, Sorbitol, Propylenglycol, Ethoxydiglycol, Aloe vera, Konjac-Manane, Hyaluronsäure, Squalan und einem pH-Wert zwischen 4,0 bis 7,0 aufweist.

19. Pharmazeutische Zusammensetzung mit hautentquellender Wirkung gemäß irgendeinem der vorangehenden Ansprüche 1 bis 18 in Form von Gel, Lipogel, Creme, Salbe, Lotion, Schaum, mit und ohne Treibmittel oder Druckgas.

20. Verwendung der pharmazeutischen Zusammensetzung mit hautentquellender Wirkung gemäß irgendeinem der Ansprüche 1 bis 19 zur Herstellung eines topischen Präparats zur Prophylaxe und/oder Behandlung von Hautquellung, -ekzemen, -rötungen, -trockenheit, -einrissen, -schuppungen und -Juckreiz.
